# EUROPÄISCHE PATENTSCHRIFT

(11) **EP 2 892 990 B1**
(45) Veröffentlichungstag und Bekanntmachung des Hinweises auf die Patenterteilung: **22.08.2018**
(21) Anmeldenummer: 13756891.1
(22) Anmeldetag: 03.09.2013
(51) Int. Cl.: C11D 3/386, C11D 3/16, C12N 9/54

(54) **WASCH- ODER REINIGUNGSMITTEL MIT VERBESSERTER ENZYMLEISTUNG**
DETERGENT OR CLEANING AGENT WITH AN IMPROVED ENZYME PERFORMANCE
PRODUITS DÉTERGENTS À ACTIVITÉ ENZYMATIQUE AMÉLIORÉE

(30) Priorität: 04.09.2012 DE 102012215642
(43) Veröffentlichungstag der Anmeldung: 15.07.2015
(73) Patentinhaber: Henkel AG & Co. KGaA, 40589 Düsseldorf (DE)
(72) Erfinder: MUSSMANN, Nina, 47877 Willich (DE); EITING, Thomas, 40589 Düsseldorf (DE); BASTIGKEIT, Thorsten, 42279 Wuppertal (DE); BENDA, Konstantin, 40217 Düsseldorf (DE); HELLMUTH, Hendrik, 40237 Düsseldorf (DE)
(86) Internationale Anmeldenummer: PCT/EP2013/068178
(87) Internationale Veröffentlichungsnummer: WO 2014/037344

(56) Entgegenhaltungen:
- WO-A1-95/23221
- WO-A1-96/41859
- WO-A1-2010/092066
- WO-A1-2012/080202
- DE-A1-102009 029 513
- DE-A1-102009 045 064
- DE-A1-102010 028 951

### SEQUENCE LISTING

<110> Henkel AG & Co. KGaA
<120> Wasch- oder Reinigungsmittel mit verbesserter Enzymleistung
<130> PT031510PCT
<150> DE 102012215642
   <151> 2012-09-04
<160> 3
<170> PatentIn version 3.5
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Ser Gly Ser Gly Ser Val Ser Ser Ile Ala Gln Gly Leu Glu Trp Ala
               100                 105                 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Gly Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die Anmeldung betrifft Enyzm-haltige Reinigungsmittel und Reinigungsverfahren unter Einsatz dieser Mittel. Gegenstand dieser Anmeldung sind insbesondere Reinigungsmittel, die spezifische Proteasen in Kombination mit 4-Formylphenylboronsäure und mindestens einem weiteren Enzym enthalten und Reinigungsverfahren, in deren Verlauf diese Mittel eingesetzt werden.
Reinigungsmittel, insbesondere Reinigungsmittel für das maschinelle Geschirrspülen und die Textilreinigung, enthalten in der Regel neben den Gerüststoffen und Tensiden als weiteren reinigungsaktiven Wirkstoff ein oder mehrere Enzyme. Typische reinigungsaktive Enzyme sind die Proteasen, die Amylasen sowie Lipasen und Cellulasen.
Ein Nachteil von Protease- und Amylase-haltigen Reinigungsmitteln aus dem Stand der Technik ist deren unzureichende Lagerstabilität und deren mangelnde amylolytische Aktivität. Häufig führt die Anwesenheit von Protease zum Verlust amylolytischer Aktivität, da die Protease die Amylase inaktiviert. Das Wasch- oder Reinigungsmittel zeigt dann keine optimale Reinigungsleistung mehr. Der vorliegenden Anmeldung liegt die Aufgabe zugrunde, den genannten Nachteil zu überwinden und protease- und amylasehaltige Reinigungsmittel bereitzustellen, die ausreichend lagerstabil sind und eine verbesserte amylolytische Aktivität aufweisen.
Überraschenderweise wurde festgestellt, dass Reinigungsmittel, die spezifische Proteasen in Kombination mit 4-Formylphenylboronsäure und mindestens einem weiteren Enzym enthalten, gegenüber Reinigungsmitteln auf Basis üblicher Proteasen eine verbesserte Reinigungsleistung aufweisen. Die 4-Formylphenylboronsäure ist ein aus dem Stand der Technik bekannter Proteaseinhibitor, dessen inhibierende Wirkung in dem erteilten europäischen Patent EP 832 174 B1 (Novozymes) beschrieben wird. Reinigungsmittel, die Protease, Amylase sowie 4-Formylphenylboronsäure enthalten, werden beispielsweise in den internationalen Anmeldungen WO 2010/034736 A1 (Unilever) und WO2010/092066 (Henkel AG & Co. KGaA) offenbart. WO2012/080202 (Henkel AG & Co. KGaA) offenbart eine flüssige Waschmittelzusammensetzung enthaltend eine Proteasemutante aus B. lentus in Kombination mit ein Amylase, wobei die Zusammensetzung ausreichend lagerstabil ist und eine verbesserte Amylaseaktivität aufweist. Ein erster Gegenstand der vorliegenden Anmeldung ist daher ein Reinigungsmittel, enthaltend
a) mindestens eine Protease, die eine Aminosäuresequenz umfasst, die zu der in SEQ ID NO. 1 angegebenen Aminosäuresequenz über deren Gesamtlänge zu mindestens 95% identisch ist und in der Zählung gemäß SEQ ID NO. 1 an Position 99 die Aminosäure Glutaminsäure (E) aufweist;
b) 4-Formylphenylboronsäure
c) mindestens ein weiteres von der Protease a) verschiendenes Enzym aus der Gruppe der Amylasen, und
d) mindestens ein organisches Lösungsmittel, wobei der Gewichtsanteil des mindestens einen organischen Lösungsmittels am Gesamtgewicht des Reinigungsmittels 0,5 bis 5,0 Gew.-% beträgt.

Erfindungsgemäße Reinigungsmittel, welche die vorgenannte Kombination einer spezifischen Protease mit 4-Formylphenylboronsäure und einem weiteren Enzym enthalten sind nicht nur lagerstabil, sondern zeichnen sich im Vergleich zu Reinigungsmitteln des Standes der Technik mit gleichem Proteasegehalt (bezogen auf aktives Enzym) aber unterschiedlicher Protease durch eine höhere enzymatische Reinigungsleistung des weiteren Enzyms c) aus.

Ein erster wesentlicher Bestandteil erfindungsgemäßer Reinigungsmittel ist die Protease a).

Vorzugsweise umfasst die Protease a) eine Aminosäuresequenz, die zu der in SEQ ID NO. 1 angegebenen Aminosäuresequenz über deren Gesamtlänge zu mindestens 95% und zunehmend bevorzugt zu mindestens 95,5%, 96%, 96,5%, 97%, 97,5%, 98%, 98,5% und 99% identisch ist und in der Zählung gemäß SEQ ID NO. 1 an Position 99 die Aminosäure Glutaminsäure (E) aufweist. Eine diesbezüglich ganz besonders bevorzugte Protease ist in SEQ ID NO. 3 angegeben.

Weitere bevorzugte Proteasen sind Proteasen wie vorstehend beschrieben, die ferner an Position 211 in der Zählung gemäß SEQ ID NO. 1 die Aminosäure Leucin (L) aufweisen.

SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird. SEQ ID NO. 2 ist die Sequenz der reifen (maturen) Protease Subtilisin 309 aus Bacillus lentus.

Besonders bevorzugte erfindungsgemäße Reinigungsmittel sind dadurch gekennzeichnet, dass die Protease a) eine Aminosäuresequenz umfasst, die zu der in SEQ ID NO. 1 angegebenen Aminosäuresequenz über deren Gesamtlänge zu mindestens 99% identisch ist und in der Zählung gemäß SEQ ID NO. 1 an Position 99 in die Aminosäure Glutaminsäure (E) aufweist. Ganz besonders bevorzugt weist die Protease a) eine Aminosäuresequenz auf, die in der Zählung gemäß SEQ ID NO. 1 in den Positionen 1-98 und 100-269 SEQ ID NO. 1 entspricht und an Position 99 die Aminosäure Glutaminsäure (E) aufweist. Eine derartige Protease ist in SEQ ID NO. 3 angegeben.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Der auf aktives Protein bezogene Gewichtsanteil der Protease a) am Gesamtgewicht erfindungsgemäß bevorzugter Reinigungsmittel beträgt vorzugsweise 0,005 bis 1,0 Gew.-%, bevorzugt 0,01 bis 0,5 Gew.-% und insbesondere 0,02 bis 0,2 Gew.-%. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgte diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die erfindungsgemäßen Reinigungsmittel enthalten als zweiten wesentlichen Bestandteil 4-Formylphenylboronsäure (4-FPBA). Der Gewichtsanteil der 4-Formylphenylboronsäure am Gesamtgewicht des Reinigungsmittels beträgt vorzugsweise 0,0005 bis 2,0 Gew.-%, bevorzugt 0,001 bis 1,0 Gew.-% und insbesondere 0,01 bis 0,1 Gew.-%.

Die erfindungsgemäßen Reinigungsmittel enthalten als weiteren wesentlichen Bestandteil c) mindestens ein weiteres Enzym. Als Enzym c) einsetzbar sind beispielsweise Lipasen oder Cutinasen, insbesondere wegen ihrer Triglycerid-spaltenden Aktivitäten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Hierzu gehören beispielsweise die ursprünglich aus *Humicola lanuginosa* (*Thermomyces lanuginosus*) erhältlichen, beziehungsweise weiterentwickelten Lipasen, insbesondere solche mit einem oder mehreren der folgenden Aminosäureaustausche ausgehend von der genannten Lipase in den Positionen D96L, T213R und/oder N233R., besonders bevorzugt T213R und N233R. Des weiteren sind beispielsweise die Cutinasen einsetzbar, die ursprünglich aus *Fusarium solani pisi* und *Humicola insolens* isoliert worden sind. Einsetzbar sind weiterhin Lipasen, beziehungsweise Cutinasen, deren Ausgangsenzyme ursprünglich aus *Pseudomonas mendocina* und *Fusarium solanii* isoliert worden sind.

Die erfindungsgemäßen Mittel können auch Cellulasen oder Hemicellulasen wie Mannanasen, Xanthanlyasen, Pektinlyasen (=Pektinasen), Pektinesterasen, Pektatlyasen, Xyloglucanasen (=Xylanasen), Pullulanasen oder β-Glucanasen enthalten.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Mit besonderem Vorzug enthalten die erfindungsgemäßen Reinigungsmittel als Enzym c) mindestens eine Amylase. Eine Amylase ist ein Enzym wie einleitend beschrieben. Für Amylasen können synonyme Begriffe verwendet werden, beispielsweise 1,4-alpha-D-Glucan-Glucanohydrolase oder Glycogenase. Erfindungsgemäß bevorzugte Amylasen sind α-Amylasen. Entscheidend dafür, ob ein Enzym eine α-Amylase im Sinne der Erfindung ist, ist deren Fähigkeit zur Hydrolyse von a(1-4)-Glykosidbindungen in der Amylose der Stärke.

Beispielhafte Amylasen sind die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Desweiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Zusammenfassend sind bevorzugte erfindungsgemäße Reinigungsmittel dadurch gekennzeichnet, dass als weiteres Enyzm c) mindestens ein Enzym aus der Gruppe der Amylasen, Cellulasen, Hemicellulasen, Mannanasen, Tannasen, Xylanasen, Xanthanasen, Xyloglucanasen, β-Glucosidasen, Pektinasen, Carrageenasen, Perhydrolasen, Oxidasen, Oxidoreduktasen oder eine Lipase, sowie deren Gemische, vorzugsweise aus der Gruppe der Amylasen eingesetzt wird. Der auf aktives Protein bezogene Gewichtsanteil des Enzyms c) am Gesamtgewicht bevorzugter Reinigungsmittels beträgt vorzugsweise 0,0005 bis 1,0 Gew.-%, bevorzugt 0,001 bis 0,5 Gew.-% und insbesondere 0,002 bis 0,2 Gew.-%.

Die Reinigungsmittel können neben den zuvor beschriebenen Inhaltsstoffen reinigungsaktive Substanzen enthalten, wobei Substanzen aus der Gruppe der Tenside, Gerüststoffe, Polymere, Glaskorrosionsinhibitoren, Korrosionsinhibitoren, Duftstoffe und Parfümträger bevorzugt werden. Diese bevorzugten Inhaltsstoffe werden in der Folge näher beschrieben.

Ein bevorzugter Bestandteil der erfindungsgemäßen Reinigungsmittel sind die nichtionischen Tenside, wobei nichtionische Tenside der allgemeinen Formel R¹-CH(OH)CH₂O-(AO)_{w}-(A'O)ₓ-(A"O)_{y}-(A'''O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
- w, x, y und z für Werte zwischen 0,5 und 120 stehen, wobei x, y und/oder z auch 0 sein können bevorzugt sind.

Durch den Zusatz der vorgenannten nichtionischen Tenside der allgemeinen Formel R¹-CH(OH)CH₂O-(AO)_{w}-(A'O)ₓ-(A"O)_{y}-(A'''O)_{z}-R², nachfolgend auch als "Hydroxymischether" bezeichnet, kann überraschenderweise die Reinigungsleistung erfindungsgemäßer Enzym-haltiger Zubereitungen deutlich verbessert werden und zwar sowohl im Vergleich zu Tensid-freien System wie auch im Vergleich zu Systemen, die alternative nichtionischen Tenside, beispielsweise aus der Gruppe der polyalkoxylierten Fettalkohole enthalten.

Durch den Einsatz dieser nichtionischen Tenside mit einer oder mehreren freien Hydroxylgruppe an einem oder beiden endständigen Alkylreste kann die Stabilität der in den erfindungsgemäßen Wasch- oder Reinigungsmittelzubereitungen enthaltenen Enzyme deutlich verbessert werden.

Bevorzugt werden insbesondere solche endgruppenverschlossene poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht.
Zur Gruppe dieser nichtionischen Tenside zählen beispielsweise die C₂₋₂₆ Fettalkohol-(PO)₁-(EO)₁₅-₄₀-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest
R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der oben stehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der allgemeine Formel R¹-CH(OH)CH₂O-(AO)_{w}-R² erwiesen, in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A für einen Rest aus der Gruppe CH₂CH₂, -CH₂CH₂-CH₂, -CH₂-CH(CH₃) steht, und
- w für Werte zwischen 1 und 120, vorzugsweise 10 bis 80, insbesondere 20 bis 40 steht
   Zur Gruppe dieser nichtionischen Tenside zählen beispielsweise die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄₋₂₂ Fettalkohol-(EO)₄₀₋₈₀-2-hydroxyalkylether

Bevorzugte Reinigungsmittel sind dadurch gekennzeichnet, dass das Reinigungsmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Reinigungsmittels vorzugsweise 0,2 bis 10 Gew.-%, bevorzugt 0,4 bis 7,0 Gew.-% und insbesondere 0,6 bis 6,0 Gew.-% beträgt.

Bevorzugte erfindungsgemäße Reinigungsmittel zur Verwendung in maschinellen Geschirrspülverfahren enthalten neben den zuvor beschriebenen nichtionischen Tensiden weitere Tenside, insbesondere amphotere Tenside enthalten. Der Anteil anionischer Tenside am Gesamtgewicht dieser Reinigungsmittel ist jedoch vorzugsweise begrenzt. So sind bevorzugte maschinelle Geschirrspülmittel dadurch gekennzeichnet, dass diese bezogen auf ihr Gesamtgewicht weniger als 5, 0 Gew.-%, vorzugsweise weniger als 3,0 Gew.-%, besonders bevorzugt weniger als 2,0 Gew.-% Aniontensid enthalten. Auf den Einsatz von anionischen Tensiden in größerer Menge wirddabei insbesondere zur Vermeidung einer übermäßigen Schaumentwicklung verzichtet.

Ein weiterer bevorzugter Bestandteil erfindungsgemäßer Reinigungsmittel sind Komplexbildner. Besonders bevorzugte Komplexbildner sind die Phosphonate. Die komplexbildenden Phosphonate umfassen neben der 1-Hydroxyethan-1,1-diphosphonsäure eine Reihe unterschiedlicher Verbindungen wie beispielsweise Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Eie im Rahmen dieser Anmeldung bevorzugtes Reinigungsmittel enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden Reinigungsmittel, welche als Phosphonate 1-Hydroxyethan-1,1-diphosphonsäure (HEDP) oder Diethylentriaminpenta(methylenphosphonsäure) (DTPMP) enthalten. Selbstverständlich können die erfindungsgemäßen Reinigungsmittel zwei oder mehr unterschiedliche Phosphonate enthalten. Bevorzugte erfindungsgemäße Reinigungsmittel sind dadurch gekennzeichnet, dass das Reinigungsmittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Reinigungsmittels vorzugsweise 0,1 und 8,0 Gew.-%, bevorzugt 0,2 und 5,0 Gew.-% und insbesondere 0,5 und 3,0 Gew.-% beträgt.

Die erfindungsgemäßen Reinigungsmittel enthalten weiterhin vorzugsweise Gerüststoff. Zu den Gerüststoffe zählen dabei insbesondere die Silikate, Carbonate, organische Cobuilder und -wo keine ökologischen Vorurteile gegen ihren Einsatz bestehen- auch die Phosphate.

Unter der Vielzahl der kommerziell erhältlichen Phosphate haben die Alkalimetallphosphate unter besonderer Bevorzugung von Pentanatriumtriphosphat, Na₅P₃O₁₀ (Natriumtripolyphosphat) bzw. Pentakaliumtriphosphat, K₅P₃O₁₀ (Kaliumtripolyphosphat) für die erfindungsgemäßen Mittel die größte Bedeutung. Werden im Rahmen der vorliegenden Anmeldung Phosphate als reinigungsaktive Substanzen in dem Reinigungsmittel eingesetzt, so enthalten bevorzugte Mittel diese(s) Phosphat(e), vorzugsweise Pentakaliumtriphosphat, wobei der Gewichtsanteil des Phosphats am Gesamtgewicht des Reinigungsmittels vorzugsweise 5,0 und 40 Gew.-%, bevorzugt 10 und 30 Gew.-% und insbesondere 12 und 25 Gew.-% beträgt.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Eine weitere besonders bevorzugte Gerüstsubstanz ist die Methylglycindiessidsäure (MGDA).

Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate.

Zur Verbesserung der Reinigungsleistung und/oder zur Einstellung der Viskosität enthalten bevorzugte Reinigungsmittel mindestens ein hydrophob modifiziertes Polymer, vorzugsweise ein hydrophob modifziertes Carbonsäuregruppen-haltiges Polymer, wobei der Gewichtsanteil des hydrophob modifizierten Polymers am Gesamtgewicht des Reinigungsmittels vorzugsweise 0,1 bis 10 Gew.-%, bevorzugt zwischen 0,2 und 8,0 Gew.-% und insbesondere 0,4 bis 6,0 Gew.-% beträgt.

In Ergänzung zu den zuvor beschriebenen Gerüststoffen können in dem Reinigungsmittel reinigungsaktive Polymere enthalten sein. Der Gewichtsanteil der reinigungsaktiven Polymere am Gesamtgewicht erfindungsgemäßer maschineller Reinigungsmittel beträgt vorzugsweise 0,1 bis 20 Gew.-%, vorzugsweise 1,0 bis 15 Gew.-% und insbesondere 2,0 bis 12 Gew.-%.

Als reinigungsaktive Polymere werden vorzugsweise Sulfonsäuregruppen-haltige Polymere, insbesondere aus der Gruppe der copolymeren Polysulfonate, eingesetzt. Diese copolymeren Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, - CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für - COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₂CH₃)-.

Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₂CH₃)-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen. Der Einsatz von teil- oder vollneutralisierten sulfonsäuregruppenhaltigen Copolymeren ist erfindungsgemäß bevorzugt.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte maschinelle Geschirrspülmittel sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigen Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Reinigungsmittel, enthaltend ein Copolymer, umfassend
i) Carbonsäuregruppen-haltige Monomer(e)
ii) Sulfonsäuregruppen-haltige Monomer(e)
iii) nichtionische Monomer(e).
werden erfindungsgemäß bevorzugt. Durch den Einsatz dieser Terpolymere konnte die Klarspülleistung erfindungsgemäßer maschineller Geschirrspülmittel gegenüber vergleichbaren Geschirrspülmitteln, die Sulfopolymere ohne Zusatz nichtionischer Monomere enthalten, verbessert werden.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und *N*-(Behenyl)acrylamid oder deren Mischungen.

Der Gewichtsanteil der Sulfonsäuregruppen-haltigen Copolymere am Gesamtgewicht erfindungsgemäßer Reinigungsmittel beträgt vorzugsweise 0,1 bis 15 Gew.-%, vorzugsweise 1,0 bis 12 Gew.-% und insbesondere 2,0 bis 10 Gew.-%.

Die erfindungsgemäßen Reinigungsmittel können in den dem Fachmann bekannten Konfektionsformen, also beispielsweise in fester oder flüssiger Form aber auch als Kombination fester und flüssiger Angebotsformen vorliegen. Als feste Angebotsformen eignen sich insbesondere Pulver, Granulate, Extrudate oder Kompaktate, insbesondere Tabletten. Die flüssigen Angebotsformen auf Basis von Wasser und/oder organischen Lösungsmitteln können verdickt, in Form von Gelen vorliegen.

Die erfindungsgemäßen Reinigungsmittel liegen vorzugsweise in flüssiger Form vor. Bevorzugte Reinigungsmittel enthalten bezogen auf ihr Gesamtgewicht mehr als 40 Gew.-%, vorzugsweise zwischen 50 und 90 Gew.-% und insbesondere zwischen 60 und 80 Gew.-% Wasser.

Als einen weiteren Bestandteil enthalten die erfindungsgemäßen Reinigungsmittel ein organisches Lösungsmittel. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Etheylenglykolmonon-butylether, Diethylenglykolmethylether, Di-ethylenglykolethylether, Propylenglykolmethyl-, -ethyl- oder -propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel. Der Gewichtsanteil dieser organischen Lösungsmittel am Gesamtgewicht erfindungsgemäßer Reinigungsmittel beträgt insbesondere 0,5 bis 5,0

Gew.-%. Ein besonders bevorzugtes und in Bezug auf die Stabilisierung der Reinigungsmittel besonders wirksames organisches Lösungsmittel ist das Glycerin sowie das 1,2 Propylenglykol. Flüssige Reinigungsmittel die mindestens ein Polyol, vorzugsweise aus der Gruppe Glycerin und 1,2-Propylenglycol enthalten, wobei der Gewichtsanteil des Polyols am Gesamtgewicht des Reinigungsmittels vorzugsweise 0,1 und 10 Gew.-%, bevorzugt 0,2 und 8,0 Gew.-% und insbesondere 0,5 und 5,0 Gew.-% beträgt, werden erfindungsgemäß bevorzugt.
Weitere bevorzugte organische Lösungsmittel sind die organischen Amine und Alkanolamine. Die erfindungsgemäßen Reinigungsmittel enthalten diese Amine vorzugsweise in Mengen von 0,1 bis 10 Gew.-%, bevorzugt von 0,2 bis 8,0 Gew.-% und insbesondere von 0,5 bis 5,0 Gew.-%, jeweils bezogen auf ihr Gesamtgewicht. Ein besonders bevorzugtes Alkanolamin ist das Ethanolamin.

Ein weiterer bevorzugter Bestandteil der erfindungsgemäßen Reinigungsmittel ist ein Zuckeralkohol (Alditol). Die Gruppe der Alditole umfasst nichtcyclische Polyole der Formel HOCH₂[CH(OH)]ₙCH₂OH. Zu den Alditolen zählen beispielsweisee Mannit (Mannitol), Isomalt, Lactit, Sorbit (Sorbitol) und Xylit (Xylitol), Threit, Erythrit und Arabit. Als in Bezug auf die Enzymstabilität besonders vorteilhaft hat sich das Sorbitol erwiesen. Der Gewichtsanteil des Zuckeralkohols am Gesamtgewicht des Reinigungsmittels beträgt vorzugsweise 1,0 bis 10 Gew.-%, bevorzugt 2,0 bis 8,0 Gew.-% und insbesondere 3,0 bis 6,0 Gew.-%.

Erfindungsgemäße flüssige Reinigungsmittel werden vorzugsweise in mehrphasiger Form, das heißt durch Kombination von zwei oder mehr voneinander getrennten unterschiedlichen flüssigen Reinigungsmitteln konfektioniert. Diese Art der Konfektionierung erhöht die Stabilität des Reinigungsmittels und verbessert dessen Reinigungsleistung. Ein erfindungsgemäß bevorzugtes Reinigungsmittel ist dadurch gekennzeichnet, dass es ein Verpackungsmittel und zwei in diesem Verpackungsmittel befindlichen voneinander getrennte flüssige Reinigungsmittel A und B umfasst, wobei die Zusammensetzung A
a) mindestens eine Protease, die eine Aminosäuresequenz umfasst, die zu der in SEQ ID NO. 1 angegebenen Aminosäuresequenz über deren Gesamtlänge zu mindestens 80% identisch ist und in der Zählung gemäß SEQ ID NO. 1 an Position 99 die Aminosäure Glutaminsäure (E) aufweist;
b) 4-Formylphenylboronsäure
c) mindestens ein weiteres, von der Protease a) verschiedenes Enzym,
d) 10 bis 84,9 Gew.-% Gerüststoff(e);
e) 15 bis 89,9 Gew.-% Wasser; enthält und
   die Zusammensetzung B
m) 10 bis 75 Gew.% Gerüststoff(e);
n) 25 bis 90 Gew.-% Wasser;
   enthält.

Die Zusammensetzung einiger bevorzugter Reinigungsmittel kann den folgenden Tabellen entnommen werden (Angaben in Gew.-% bezogen auf das Gesamtgewicht des Reinigungsmittels sofern nicht anders angegeben).

| | Formel 1 | Formel 2 | Formel 3 | Formel 4 | Formel 5 |
|---|---|---|---|---|---|
| Protease a)* | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Enzym c)** | 0,0005 bis 1,0 | 0,001 bis 0,5 | 0,002 bis 0,2 | 0,004 | 0,012 |
| Misc | add 100 | add 100 | add 100 | add 100 | add 100 |

| | | | | | |
|---|---|---|---|---|---|
| * Protease gemäß Anspruch 1, Merkmal a) ** von der Protease a) verschiedenes Enzym | | | | | |

| | Formel 6 | Formel 7 | Formel 8 | Formel 9 | Formel 10 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Misc | add 100 | add 100 | add 100 | add 100 | add 100 |

| | | | | | |
|---|---|---|---|---|---|
| * Protease gemäß Anspruch 2 | | | | | |

| | Formel 11 | Formel12 | Formel 13 | Formel 14 | Formel 15 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Wasser | > 40 | 50 bis 85 | 60 bis 80 | 64 | 71 |
| Misc | add 100 | add 100 | add 100 | add 100 | add 100 |

| | | | | | |
|---|---|---|---|---|---|
| * Protease gemäß Anspruch 2 | | | | | |

| | Formel 16 | Formel 17 | Formel 18 | Formel 19 | Formel 20 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Gerüststoff | 5,0 bis 40 | 10 bis 30 | 12 bis 25 | 26 | 18 |
| Wasser | > 40 | 50 bis 85 | 60 bis 80 | 64 | 71 |
| Misc | add 100 | add 100 | add 100 | add 100 | add 100 |

| | | | | | |
|---|---|---|---|---|---|
| * Protease gemäß Anspruch 2 | | | | | |

| | Formel 21 | Formel 22 | Formel 23 | Formel 24 | Formel 25 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Niotensid | 0,2 bis 10 | 0,4 bis 7,0 | 0,6 bis 6,0 | 4,0 | 2,0 |
| Wasser | > 40 | 50 bis 85 | 60 bis 80 | 64 | 71 |
| Misc | add 100 | add 100 | add 100 | add 100 | add 100 |

| | | | | | |
|---|---|---|---|---|---|
| * Protease gemäß Anspruch 2 | | | | | |

| | Formel 26 | Formel 27 | Formel 28 | Formel 29 | Formel 30 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Gerüststoff | 5,0 bis 40 | 10 bis 30 | 12 bis 25 | 26 | 18 |
| Niotensid | 0,2 bis 10 | 0,4 bis 7,0 | 0,6 bis 6,0 | 4,0 | 2,0 |
| Wasser | > 40 | 50 bis 85 | 60 bis 80 | 64 | 71 |
| Misc | add 100 | add 100 | add 100 | add 100 | add 100 |

| | | | | | |
|---|---|---|---|---|---|
| * Protease gemäß Anspruch 2 | | | | | |

| | Formel 31 | Formel 32 | Formel 33 | Formel 34 | Formel 35 |
|---|---|---|---|---|---|
| Protease a) * | 0,005 bis 1,0 | 0,01 bis 0,5 | 0,02 bis 0,2 | 0,06 | 0,17 |
| 4-FPBA | 0,0005 bis 2,0 | 0,001 bis 1,0 | 0,01 bis 0,1 | 0,02 | 0,04 |
| Amylase | 0,0005 bis 1,0 | 0,001 bis 0,5 | 0,002 bis 0,2 | 0,004 | 0,012 |
| Pentakaliumtriphosphat | 5,0 bis 40 | 10 bis 30 | 12 bis 25 | 18 | 12 |
| HEDP | 0,1 bis 8,0 | 0,2 bis 5,0 | 0,5 bis 3,0 | 3,0 | 2,0 |
| Sulfo-Copolymer | 0,1 bis 15 | 1,0 bis 12 | 2,0 bis 10 | 4,0 | 6,0 |
| Hydroxymischether | 0,2 bis 10 | 0,4 bis 7,0 | 0,6 bis 6,0 | 4,0 | 2,0 |
| Wasser | > 40 | 50 bis 85 | 60 bis 80 | 64 | 71 |
| Misc | add 100 | add 100 | add 100 | add 100 | add 100 |

| | | | | | |
|---|---|---|---|---|---|
| * Protease gemäß Anspruch 2 | | | | | |

Ein zweiter Gegenstand der vorliegenden Anmeldung ist ein Reinigungsverfahren unter Einsatz eines der zuvor beschriebenen erfindungsgemäßen Reinigungsmittel. In einer bevorzugten Ausführungsform erfindungsgemäßer Verfahren wird das Reinigungsmittel im Verlauf des Verfahrens in eine wässrige Reinigungsflotte eingetragen.

Ein bevorzugtes Reinigungsverfahren ist ein maschinelles Geschirrspülverfahren. Die Dosierung des erfindungsgemäßen Reinigungsmittels in die Reinigungsflotte kann in einem solchen Verfahren beispielsweise mittels der Dosierkammer in der Tür oder mittels eines zusätzlichen Dosierbehälters im Innenraum der Geschirrspülmaschine erfolgen. Alternativ kann das Reinigungsmittel auch direkt auf das verschmutzte Geschirr oder auf eine der Innenwände der Geschirrspülmaschine, beispielsweise die Innenseite der Tür aufgebracht werden.

Die Durchführung des erfindungsgemäßen Verfahrens erfolgt im Innenraum einer handelsüblichen Geschirrspülmaschine. Das Reinigungsprogramm kann bei einer Geschirrspülmaschine in der Regel vor Durchführung des Geschirrspülverfahrens durch den Verbraucher gewählt und festgelegt werden. Das in dem erfindungsgemäßen Verfahren eingesetzte Reinigungsprogramm der Geschirrspülmaschine umfasst dabei mindestens einen Vorspülgang und einen Reinigungsgang. Erfindungsgemäß bevorzugt werden Reinigungsprogramme, die weitere Reinigungs- oder Spülgänge, beispielsweise einen Klarspülgang umfassen. Das erfindungsgemäße Verfahren ist mit besonderem Vorzug Bestandteil eines Reinigungsprogramms, umfassend einen Vorspülgang, einen Reinigungsgang sowie einen Klarspülgang. Das erfindungsgemäße Verfahren wird bevorzugt in Verbindung mit solchen Reinigungsprogrammen eingesetzt, bei denen die Waschflotte im Verlauf des Reinigungsgangs erwärmt wird. In einer bevorzugten Ausführungsform des erfindungsgemäßen Verfahrens ist der Reinigungsgang, in dessen Verlauf das erfindungsgemäße Reinigungsmittel in den Innenraum der Geschirrspülmaschine eindosiert wird dadurch gekennzeichnet, dass in seinem Verlauf die Temperatur der Reinigungsflotte auf Werte oberhalb 30°C, vorzugsweise oberhalb 40°C und insbesondere oberhalb 50°C ansteigt.

Bevorzugte Ausführungsformen des erfindungsgemäßen maschinellen Geschirrspülverfahrens ergeben sich mutatis mutandis aus der bisherigen Beschreibung bevorzugter Ausführungsformen des erfindungsgemäßen Reinigungsmittels, auf die zur Vermeidung von Wiederholungen an dieser Stelle verwiesen wird.

Ein dritter Gegenstand der vorliegenden Anmeldung ist die Verwendung von 4-Formylphenylboronsäure in Reinigungsmitteln, enthaltend
a) mindestens eine Protease, die eine Aminosäuresequenz umfasst, die zu der in SEQ ID NO. 1 angegebenen Aminosäuresequenz über deren Gesamtlänge zu mindestens 95% identisch ist und in der Zählung gemäß SEQ ID NO. 1 an Position 99 die Aminosäure Glutaminsäure (E) aufweist;
   sowie
b) mindestens ein weiteres von der Protease a) verschiedenes Enzym, vorzugsweise mindestens eine weitere Amylase, und
c) mindestens ein organisches Lösungsmittel, wobei der Gewichtsanteil des mindestens einen organischen Lösungsmittels am Gesamtgewicht des Reinigungsmittels 0,5 bis 5,0 Gew.-% beträgt,
zur Steigerung der enzymatischen Reinigungsleistung, vorzugsweise der amylolytischen Reinigungsleistung des Reinigungsmittels.

Die erfindungsgemäße Verwendung erfolgt vorzugsweise zur Entfernung von amylase-sensitiven Anschmutzungen auf Textilien oder harten Oberflächen. Besonders bevorzugt ist es, die erfindungsgemäßen Reinigungsmittels als maschinelle Geschirrspülmittel zu verwenden.

### Beispiel: Ermittlung der Reinigungsleistung erfindungsgemäßer maschineller Geschirrspülmittel

Als Basisrezeptur diente ein zweiphasiges flüssiges maschinelles Geschirrspülmittel der folgenden Zusammensetzung (alle Angaben in Gewichts-Prozent):
(a) Enzymphase:

| | |
|---|---|
| Builder | 18,0 |
| Zuckeralkohol | 12,0 |
| Nichtionisches Tensid (C8-C10 Fettalkoholethoxylat mit 22 EO) | 5,0 |
| Alkaliverbindung (Base) | 3,5 |
| Borsäure | 3,0 |
| Phosphonat (HEDP) | 1,5 |
| Amylase-Zubereitung | 1,5 |
| Protease-Zubereitung | 3,5 |
| 4-FPBA | 0,02 |
| Ca-Salz | 1,2 |
| Zn-Salz | 0,2 |
| Verdicker | 1,0 |
| Farbstoff, Parfüm, Konservierungsmittel | 0,3 |
| Wasser | ad 100 |

(b) Alkalische Phase:

| | |
|---|---|
| Builder | 12,0 |
| Natriumcarbonat | 10,0 |
| Sulfopolymer | 7,0 |
| Alkaliverbindung (Base) | 4,0 |
| Monoethanolamin | 3,5 |
| Phosphonat (HEDP) | 4,0 |
| Verdicker | 1,0 |
| Farbstoff, Parfüm, Konservierungsmittel | 0,3 |
| Wasser | ad 100 |

Die Enzymphase der Basisrezeptur wurde für die verschiedenen Versuchsansätze jeweils mit 3,5 Gew.-% von Präparationen der folgenden Proteasen versetzt:
V1: Savinase Ultra 16 L (Novozymes)
E1: Protease, die eine Aminosäuresequenz aufweist, die in der Zählung gemäß SEQ ID NO. 1 in den Positionen 1-98 und 100-269 SEQ ID NO. 1 entspricht und an Position 99 die Aminosäure Glutaminsäure (E) aufweist;

Zur Ermittlung der Reinigungsleistung wurden beide Phasen zu gleichen Teilen dosiert (jeweils 20g pro Phase). Gewaschen wurde in einem pH-Wertebereich zwischen pH 9 und pH 10 in einer Geschirrspülmaschine G698SC der Firma Miele in einem Volumen von 5 Litern für eine Dauer von 60 Minuten bei einer Temperatur von 50°C. Es wurde Geschirr mit Haferflocken-Anschmutzungen verwendet.

Die Auswertung der Reinigungsleistung erfolgt gemäß der Standard IKW-Methode visuell anhand einer Skala von 1 bis 10, wobei der Wert 10 die beste Note ist (kein erkennbarer Rückstand). Die Ergebnisse sind in nachstehender Tabelle 1 zusammengefasst:

**Tabelle 1:**

| | IKW-Note |
|---|---|
| V1 | 6,5 |
| E1 | 7,3 |

Die Versuchsreihe zeigt, dass die erfindungsgemäßen Zusammensetzung E1 gegenüber der Vergleichsrezeptur des Standes der Technik eine deutlich verbesserte amylolytische Reinigungsleistung aufweist.

## Patentansprüche

1. Reinigungsmittel, enthaltend
a) mindestens eine Protease, die eine Aminosäuresequenz umfasst, die zu der in SEQ ID NO. 1 angegebenen Aminosäuresequenz über deren Gesamtlänge zu mindestens 95% identisch ist und in der Zählung gemäß SEQ ID NO. 1 an Position 99 die Aminosäure Glutaminsäure (E) aufweist;
b) 4-Formylphenylboronsäure,
c) mindestens ein weiteres von der Protease a) verschiedenes Enzym aus der Gruppe der Amylasen, und
d) mindestens ein organisches Lösungsmittel, wobei der Gewichtsanteil des mindestens einen organischen Lösungsmittels am Gesamtgewicht des Reinigungsmittels 0,5 bis 5,0 Gew.-% beträgt.

2. Reinigungsmittel nach Anspruch 1, **dadurch gekennzeichnet, dass** die Protease a1) eine Aminosäuresequenz aufweist, die in der Zählung gemäß SEQ ID NO. 1 in den Positionen 1-98 und 100-269 SEQ ID NO. 1 entspricht und an Position 99 die Aminosäure Glutaminsäure (E) aufweist.

3. Reinigungsmittel nach einem der vorherigen Ansprüche, **dadurch gekennzeichnet, dass** der Gewichtsanteil der Protease am Gesamtgewicht des Reinigungsmittels bezogen auf aktives Protein 0,005 bis 1,0 Gew.-%, bevorzugt 0,01 bis 0,5 Gew.-% und insbesondere 0,02 bis 0,2 Gew.-% beträgt.

4. Reinigungsmittel nach einem der vorherigen Ansprüche, **dadurch gekennzeichnet, dass** der Gewichtsanteil der 4-Formylphenylboronsäure am Gesamtgewicht des Reinigungsmittels 0,0005 bis 2,0 Gew.-%, vorzugsweise 0,001 bis 1,0 Gew.-% und insbesondere 0,01 bis 0,1 Gew.-% beträgt.

5. Reinigungsmittel nach einem der vorherigen Ansprüche, **dadurch gekennzeichnet, dass** der Gewichtsanteil des Enzyms c) am Gesamtgewicht des Reinigungsmittels bezogen auf aktives Protein 0,0005 bis 1,0 Gew.-%, bevorzugt 0,001 bis 0,5 Gew.-% und insbesondere 0,002 bis 0,2 Gew.-% beträgt.

6. Reinigungsverfahren unter Einsatz eines Reinigungsmittels nach einem der Ansprüche 1 bis 5.

7. Verwendung von 4-Formylphenylboronsäure in Reinigungsmitteln, enthaltend
a) mindestens eine Protease, die eine Aminosäuresequenz umfasst, die zu der in SEQ ID NO. 1 angegebenen Aminosäuresequenz über deren Gesamtlänge zu mindestens 95% identisch ist und in der Zählung gemäß SEQ ID NO. 1 an Position 99 die Aminosäure Glutaminsäure (E) aufweist;
sowie
b) mindestens ein weiteres von der Protease a) verschiedenes Enzym, vorzugsweise mindestens eine weitere Amylase, und
c) mindestens ein organisches Lösungsmittel, wobei der Gewichtsanteil des mindestens einen organischen Lösungsmittels am Gesamtgewicht des Reinigungsmittels 0,5 bis 5,0 Gew.-% beträgt.
zur Steigerung der enzymatischen Reinigungsleistung, vorzugsweise der amylolytischen Reinigungsleistung des Reinigungsmittels.

## Claims

1. A cleaning agent, containing
a) at least one protease that has an amino acid sequence which is at least 95% identical over the total length thereof to the amino acid sequence specified in SEQ ID NO. 1 and has the amino acid glutamic acid (E) at position 99 using the numbering according to SEQ ID NO. 1;
b) 4-formylphenylboronic acid,
c) at least one further enzyme that is different from the protease a) and is from the group of amylases, and
d) at least organic solvent, wherein the weight proportion of the at least one organic solvent with respect to the total weight of the cleaning agent is 0.5 to 5.0 wt.%.

2. The cleaning agent according to claim 1, **characterized in that** the protease a1) has an amino acid sequence which corresponds to SEQ ID NO. 1 in the positions 1-98 and 100-269 using the numbering according to SEQ ID NO. 1 and has the amino acid glutamic acid (E) at position 99.

3. The cleaning agent according to one of the preceding claims, **characterized in that** the weight proportion of the protease with respect to the total weight of the cleaning agent is 0.005 to 1.0 wt.%, preferably 0.01 to 0.5 wt.% and in particular 0.02 to 0.2 wt.%, based on active protein.

4. The cleaning agent according to one of the preceding claims, **characterized in that** the weight proportion of 4-formylphenylboronic acid with respect to the total weight of the cleaning agent is 0.0005 to 2.0 wt.%, preferably 0.001 to 1.0 wt.% and in particular 0.01 to 0.1 wt.%.

5. The cleaning agent according to one of the preceding claims, **characterized in that** the weight proportion of the enzyme c) with respect to the total weight of the cleaning agent is 0.0005 to 1.0 wt.%, preferably 0.001 to 0.5 wt.% and in particular 0.002 to 0.2 wt.%, based on active protein.

6. A cleaning method using a cleaning agent according to one of claims 1 to 5.

7. The use of 4-formylphenylboronic acid in cleaning agents, containing
a) at least one protease that has an amino acid sequence which is at least 95% identical over the total length thereof to the amino acid sequence specified in SEQ ID NO. 1 and has
the amino acid glutamic acid (E) at position 99 using the numbering according to SEQ ID NO. 1;
and
b) at least one further enzyme that is different from the protease a), preferably at least one further amylase, and
c) at least organic solvent, wherein the weight proportion of the at least one organic solvent with respect to the total weight of the cleaning agent is 0.5 to 5.0 wt.%.
for enhancing the enzymatic cleaning performance, preferably the amylolytic cleaning performance of the cleaning agent.

## Revendications

1. Détergent, contenant :
a) au moins une protéase, comprenant une séquence d'acides aminés qui est identique à au moins 95 % à la séquence d'acides aminés indiquée en SEQ ID NO. 1 sur toute sa longueur et comprend l'acide aminé acide glutamique (E) à la position 99, dans le comptage selon SEQ ID NO. 1 ;
b) l'acide 4-formylphénylboronique,
c) au moins une autre enzyme différente de la protéase a) issue de l'ensemble des amylases, et
d) au moins un solvant organique, la proportion en poids de l'au moins un solvant organique dans le poids total du détergent représentant 0,5 à 5,0 % en poids.

2. Détergent selon la revendication 1, **caractérisé en ce que** la protéase a1) comprend une séquence d'acides aminés qui correspond à SEQ ID NO.1, aux positions 1 à 98 et 100 à 269 dans le comptage selon SEQ ID NO. 1, et comprend l'acide aminé acide glutamique (E) à la position 99.

3. Détergent selon l'une des revendications précédentes, **caractérisé en ce que** la proportion en poids de la protéase dans le poids total du détergent par rapport à une protéine active représente 0,005 à 1,0 % en poids, de préférence 0,01 à 0,5 % en poids et en particulier 0,02 à 0,2 % en poids.

4. Détergent selon l'une des revendications précédentes, **caractérisé en ce que** la proportion en poids de l'acide 4-formylphénylboronique dans le poids total du détergent représente 0,0005 à 2,0 % en poids, de préférence 0,001 à 1,0 % en poids et en particulier 0,01 à 0,1 % en poids.

5. Détergent selon l'une des revendications précédentes, **caractérisé en ce que** la proportion en poids de l'enzyme c) dans le poids total du détergent par rapport à une protéine active représente 0,0005 à 1,0 % en poids, de préférence 0,001 à 0,5 % en poids et en particulier 0,002 à 0,2 % en poids.

6. Procédé de nettoyage utilisant un détergent selon l'une des revendications 1 à 5.

7. Utilisation d'acide 4-formylphénylboronique dans des détergents contenant
a) au moins une protéase, comprenant une séquence d'acides aminés qui est identique à au moins 95 % à la séquence d'acides aminés indiquée en SEQ ID NO. 1 sur toute sa longueur et comprend l'acide aminé acide glutamique (E) à la position 99, dans le comptage selon SEQ ID NO. 1 ;
ainsi que
b) au moins une autre enzyme différente de la protéase a), de préférence au moins une autre amylase, et
c) au moins un solvant organique, la proportion en poids de l'au moins un solvant organique dans le poids total du détergent représentant 0,5 à 5,0 % en poids,
afin d'augmenter le pouvoir nettoyant enzymatique, de préférence le pouvoir nettoyant amylolytique du détergent.
